# EUROPEAN PATENT SPECIFICATION

(11) **EP 2 440 917 B1**
(45) Date of publication and mention of the grant of the patent: **15.05.2013**
(21) Application number: 10728916.7
(22) Date of filing: 10.06.2010
(51) Int. Cl.: G01N 33/08, A01K 43/08, B07C 5/34, G01G 9/00, G01G 11/00, G01N 23/04

(54) **METHOD FOR DETERMINING WEIGHTS OF EGGS**
VERFAHREN ZUR GEWICHTSBESTIMMUNG BEI EIERN
PROCÉDÉ PERMETTANT DE DÉTERMINER LE POIDS D'OEUFS

(30) Priority: 11.06.2009 EP 09007728
(43) Date of publication of application: 18.04.2012
(73) Proprietor: Moba Group B.V., 3771 VE Barneveld (NL)
(72) Inventor: DE BAERDEMAEKER, Josse, B - 3001 Leuven (BE); KEMPS, Bart, B - 3001 Leuven (BE); DE KRIJGER, Marjo, NL-3815 MN Amersfoort (NL); DE KETELAERE, Bart, B - 3001 Leuven (BE); WOITTIEZ, Michiel, B - 3001 Leuven (BE); DOORNEKAMP, Martin, NL-3863 DZ Nijkerk (NL)
(74) Representative: Jansen, Cornelis Marinus
(86) International application number: PCT/NL2010/050353
(87) International publication number: WO 2010/143953

(56) References cited:
- NL-C1- 1 018 940
- US-A1- 2002 189 321
- US-A1- 2007 278 284
- DATABASE WPI Week 200749 Thomson Scientific, London, GB; AN 2007-498570 XP002603665 & CN 1 934 935 A (UNIV ZHEJIANG) 28 March 2007 (2007-03-28)

## Description

The present invention relates to a method for determining weights of eggs, comprising
- exposing the eggs to electromagnetic radiation having a wavelength of at least 200 nm and thereby illuminating and/or candling the eggs,
- recording and processing images that are obtained from differences in intensity of radiation transmitted during illuminating and/or candling, whereby image data and/or derivatives thereof are established.

In 'Wiskundig broeden op een ei', A. Heck, 2004, it is explained at length how the volume of eggs can be determined from photographs of these eggs. Besides an explanation of the mathematical modeling of the volume of an egg, an egg diameter is determined from a photograph in a generally known manner, whereupon furthermore, in accordance with considerations of symmetry, the egg volume (V) is determined. With this, in a manner known to one skilled in the art, the egg weight (or also the egg mass (m)) can be determined by combining the volume with values, for example known from the literature, for the density (p), or also the average density, according to m = p.V.

This manner of weight determination cannot be simply applied in the art of sorting eggs, because greater accuracies are required there than can be obtained with density values as mentioned above.

In EP1856971 a method and apparatus are described whereby newly-laid and then collected eggs are counted. These counts are carried out on the basis of image recognition with cameras, in the context of which especially the avoidance of double counts is elucidated. In a further elaboration of this image recognition, determining the weight of a counted egg is mentioned. With the aid of these determinations, overviews of the weights of the counted eggs can be compiled, allowing the status of the processes in the poultry houses to be monitored better and control of the poultry houses to be improved.

Such a method and apparatus can be used for determining the weights of passing eggs, and hence for further sorting of these eggs. As is generally known, eggs are sorted, *inter alia* by weight, where especially the exact weight distribution of these eggs, more specifically their distribution over the well-known weight classes S-M-L-XL, etc., is of crucial importance. More particularly, a sophisticated division of the eggs by their weights precisely around the limits of these weight classes is of great importance. This importance is directly related to the financial proceeds of a batch of eggs.

Determining the weights according to EP1856971 is not sufficient for precision sorting as described above.

To enhance the accuracy of the weight determination, the method according to the present invention is characterized in that the method furthermore comprises at least a second type of detection, wherein in combination with the above-mentioned image data and/or derivatives thereof the weight of each egg is obtained.

Aspects of the invention concern, for example, utilization of a second type of measurement, for taking into account details of the eggshell (for example, shell thickness) and/or of the egg air cell, to meet the need for enhanced accuracy, which will be further elucidated below.

The invention can also be defined as follows:
method for determining weights of eggs, comprising
   - using a first type of egg detection, comprising making images of the eggs utilizing electromagnetic radiation having a wavelength of at least 200 nm;
   - using a second type of detection to detect the eggs; and
   - processing detection results of both the first type of detection and the second type of detection for determining the weights.

Detection results of the first type of detection comprise in particular the images of the eggs (in particular, images obtained from differences in intensity of radiation transmitted in illuminating and/or candling), for example image data. The term 'detection results' should herein be understood to include 'derivatives of detection results'.

Detection results of the second type of detection depend on the kind of detection, and comprise, for example, stiffness in the case of stiffness detection, or images of an air cell and/or eggshell obtained with X-ray radiation in the case of X-ray detection.

More particularly, exemplary embodiments of this invention have one or more of the following features:
that the second type of detection comprises determining at least the stiffness K and/or derivatives thereof of the eggshell of each egg as a result of a pressure force changing in time exerted on the eggshell;
that such an egg is caused to vibrate with a tapper unit, wherein at least the stiffness K and/or derivatives thereof are determined from the vibration characteristics of the egg, wherein thereupon the weight of the egg is determined by combining the image data and/or derivatives thereof (i.e., measuring results of the first type of detection concerning the egg) with the shell stiffness and/or the derivatives thereof.

Furthermore, a further elaboration of the invention comprises a second type of detection wherein such egg is caused to vibrate with a tapper unit, wherein at least the stiffness K and/or derivatives thereof are determined from the vibration characteristics of the tapper unit, wherein thereupon the weight of the egg is determined by combining the image data and/or derivatives thereof (i.e., measuring results of the first type of detection concerning the egg) with the shell stiffness and/or the derivatives thereof.

Since in the current egg sorting machines eggs are practically always tested for breakage with the aid of vibration analysis, the exemplary embodiments mentioned above have the great advantage that the same analysis data can be used for weight determination. Furthermore, as a result, detectors for weighing, viz. in general weighbridge devices such as load cells, can be omitted, whereby a further possibility of pollution is obviated and hence an improvement of hygiene is obtained.

In particular, it is known in this field of technology to test the shells of the eggs by subjecting them to a controlled pressure force. In EP738888 the eggs are tapped with a tapper of which a bouncing ball body generates signals that are indicative of the local eggshell status of such an egg. According to EP1238582 the eggshells are also tested, but in contrast to EP738888 the vibration modes of the eggs as a whole are mapped. In NL1018940 it is explained how from the obtained vibration characteristics specific mechanical properties of these eggs can be derived.

In other exemplary embodiments, the method according to the invention has one or more of the features:
that the second type of detection comprises exposing the eggs to X-rays whereby at least data of the air cell of each egg are obtained, wherein the weight (of the egg) is determined by combining the image data and/or derivatives thereof (i.e., measuring results of the first type of detection) with the data of the air cell; and/or
that the second type of detection comprises exposing the eggs to X-rays whereby at least data of the eggshell of each egg are obtained, wherein the weight is determined by combining the image data and/or derivatives thereof (i.e., measuring results of the first type of detection) with the data of the eggshell,
that the X-rays have a wavelength of at most 5 nm for therewith candling the eggs,
   - wherein images are recorded of differences in intensity of radiation transmitted during candling, and
   - wherein these images are processed whereby at least transitions and/or features derived therefrom in substantially density are established;
that the X-ray radiation comprises substantially radiation of the line spectrum type;
that the X-ray radiation comprises substantially radiation of the continuous spectrum type; and/or
that the X-ray radiation comprises radiation of both the continuous spectrum and of the line spectrum type.

With great advantage, the above-defined method can be used with generally known apparatuses for medical applications of X-rays. These are understood to include, for example, units as set up for dental checks, these units being built into very compact housings.

In particular, X-rays are used in WO2010074572 also in applicant's name, in which also with X-rays details of the structure of the egg are established to thereby enable precision determinations of the weight of such an egg. That apparatus and method, however, cannot be used for quick measurements. In addition, the invention relates to a method for classifying eggs, comprising,
- using the method according to at least one of the preceding definitions, and
- comparing at least the weights established according to the foregoing with criteria drawn up for classifying.

Furthermore, the method according to the invention relates to the sorting of eggs, comprising,
- classifying eggs as defined above, and
- in accordance with the classifying, sorting the eggs, wherein the eggs are collected in packing units, and has as a further feature that the eggs are located on rotating rollers of an egg sorting machine.

Hereinbelow, the invention will be described in detail with reference to a drawing, with
Fig. 1, as a diagram for the method according to the present invention, and
Fig. 2, with an X-ray recording made with a generally known camera for medical applications.

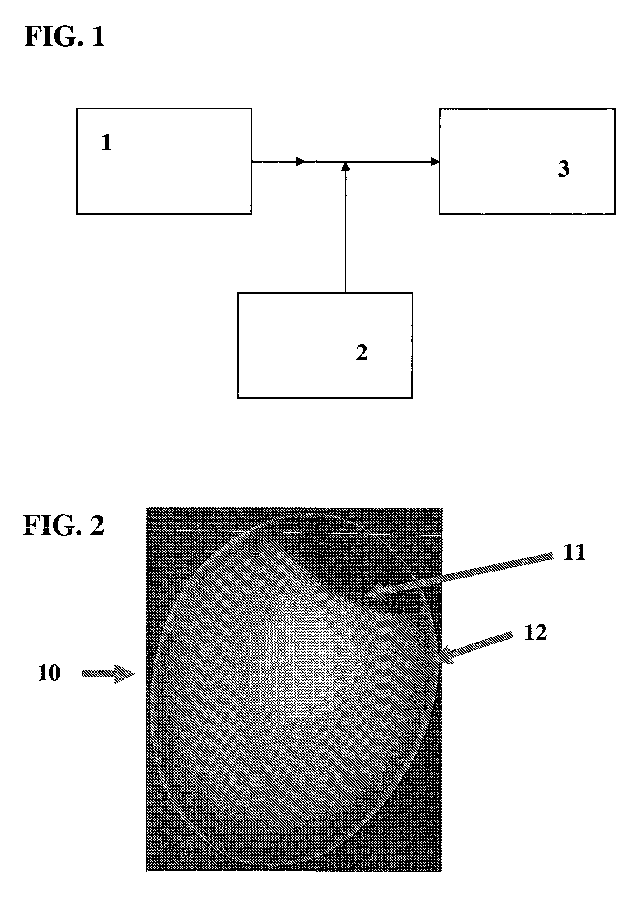

Fig. 1 shows schematically an example of a method for determining weights of eggs, comprising (step 1) using a first type of egg detection, comprising making images of the eggs utilizing electromagnetic radiation having a wavelength of at least 200 nm; (step 2) using a second type of detection to detect the eggs; and (step 3) processing detection results of both the first type of detection and the second type of detection for determining the weights.

Fig. 1 is thus a highly schematic representation of the consecutive steps according to the present method for determining - in a different manner than mechanically - the weight or the mass of an egg. To avoid any confusion, it is noted here that mass and weight herein have the same meaning, since in the use of the present technology, physically speaking, no special circumstances occur that could necessitate making a distinction between mass and weight. The mass, or the weight, will be designated, if necessary, with the symbol 'm'.

In Fig. 1 the first step of the method according to the invention is represented with block 1. This step involves optically imaging an egg. Optically is understood to mean the use of the electromagnetic spectrum in substantially the visible region, in particular at wavelengths from 200 nm. Imaging will be understood to mean to observe the object, that is, the egg, with the observation being recorded in a manner generally known to one skilled in the art.

Recording will generally involve a camera but can also comprise scanning with a beam. In all these cases the observation will make it possible to determine the circumference. This circumference - in view of the fact that in the great majority of cases the shape of the egg is practically symmetrical, viz. symmetrical around the long axis of the egg - will make it possible to determine the volume of the egg in a simple manner. There where the deviation from the symmetry proves too large, the circumference, for example as a contour, is used for further determinations.

As already indicated above, thereupon the mass or the weight can be determined in a generally known manner, i.e., utilizing measuring results, for example an egg circumference, from the first type of detection. As mentioned, it holds then that in a generally known manner the egg volume (V) is determined from the egg circumference (egg diameter). With this, the egg weight (or also the egg mass (m)) can be determined by combining the volume with values, for example known from literature, for the density (p), or also the average density, according to m = p.V.

As described hereinbefore in the introduction, this determination is insufficient for the above-mentioned application of accurate sorting. To remedy this deficiency, in the diagram a block 2 is shown which represents a second step in the method according to the invention. This second step is a second detection whereby the deficient accuracy of the first detection is met. With it, in particular, further details of the egg body, viz. the air cell and/or the eggshell, will be determined. Further, it has been found that differences in compositions of substantially egg fluid substantially formed by the egg white and the egg yolk are not relevant to the above-mentioned accuracy required for sorting.

It has been found that this second step (2) can be carried out in several different ways. A first possibility is to determine the vibration properties of such an egg. More particularly, the stiffness K can be determined. This possibility has already been elucidated hereinabove in the discussion of NL1018940. Wholly different from the dynamic properties of such a physical body is one of the derived properties, the thickness of the eggshell.

As mentioned, the egg may for example be caused to vibrate with a tapper unit, with at least the stiffness K and/or derivatives thereof being determined from the vibration characteristics of the egg. Also, the egg may be caused to vibrate with a tapper unit, with at least the stiffness K and/or derivatives thereof being determined from the vibration characteristics of the tapper unit.

As follows from NL1018940, with the stiffness K the thickness T of the eggshell can be determined. With this, as indicated schematically with block 3 in Fig. 1, in combination with the determinations according to block 1, the weight determination as elucidated above can be so adjusted that the accuracy then attained in many cases is sufficient for the application of accurate sorting.

In a further exemplary embodiment for the above-mentioned second step according to block 2, an X-ray image of such an egg is used. In particular in Fig. 2 an example is represented. The photograph shown is made with generally known camera systems for medical applications. The X-ray radiation used there will have a wavelength of at most 5 nm. This technology has meanwhile been developed so far that well-shielded cameras and quick real time data processing are possible. In this photograph of an egg 10, both the air cell 11 and the shell 12 can be seen. Also for this manner of measurement, it has been found that in combination with a volume determination according to block 1 (as described above), the geometric properties of the shell and the air cell can be determined such that with well-known reference values for densities, for example in combination with a statistic calculation model, the weight of such an egg can be determined very accurately according to the step as represented in block 3.

To one skilled in the art it will be clear that small changes and variants are understood to be covered by the scope of the appended claims. By using, for example, details of contrasts in camera pictures, correspondingly more details and refinements in the determinations of the weight can be realized.

## Claims

1. A method for determining weights of eggs, comprising,
- exposing the eggs to electromagnetic radiation having a wavelength of at least 200 nm and thereby illuminating and/or candling the eggs,
- recording and processing images that are obtained from differences in intensity of radiation transmitted during illuminating and/or candling, whereby image data and/or derivatives thereof are established,
**characterized in that**
the method furthermore comprises at least a second type of detection, wherein in combination with said image data and/or derivatives thereof the weight of each egg is obtained.

2. A method according to claim 1, **characterized in that** said second type of detection comprises determining at least the stiffness K and/or derivatives thereof of the eggshell of each egg as a result of a pressure force changing in time exerted on said eggshell.

3. A method according to claim 2, **characterized in that** such an egg is caused to vibrate with a tapper unit, wherein at least said stiffness K and/or derivatives thereof are determined from the vibration characteristics of said egg, wherein thereupon the weight is determined by combining said image data and/or derivatives thereof with said shell stiffness and/or said derivatives thereof.

4. A method according to claim 2, **characterized in that** such an egg is caused to vibrate with a tapper unit, wherein at least said stiffness K and/or derivatives thereof are determined from the vibration characteristics of said tapper unit, wherein thereupon the weight is determined by combining said image data and/or derivatives thereof with said shell stiffness and/or said derivatives thereof.

5. A method according to claim 1, **characterized in that** said second type of detection comprises exposing the eggs to X-rays whereby at least data of the air cell of each egg are obtained, wherein the weight is determined by combining said image data and/or derivatives thereof with said data of the air cell.

6. A method according to claim 1, **characterized in that** said second type of detection comprises exposing the eggs to X-rays whereby at least data of the eggshell of each egg are obtained, wherein the weight is determined by combining said image data and/or derivatives thereof with said data of the eggshell.

7. A method according to claim 5 or 6, **characterized in that** the X-rays have a wavelength of at most 5 nm for therewith candling the eggs,
- wherein images are recorded of differences in intensity of radiation transmitted during candling, and
- wherein these images are processed whereby at least transitions and/or features derived therefrom in substantially density are established.

8. A method according to any one of claims 5, 6, or 7, **characterized in that** the X-ray radiation comprises substantially radiation of the line spectrum type.

9. A method according to any one of claims 5, 6, or 7, **characterized in that** the X-ray radiation comprises substantially radiation of the continuous spectrum type.

10. A method according to any one of claims 5, 6, or 7, **characterized in that** the X-ray radiation comprises radiation of both the continuous spectrum and of the line spectrum type.

11. A method according to any of the preceding claims, comprising (step 1) using a first type of egg detection, comprising making the images of the eggs utilizing the electromagnetic radiation having a wavelength of at least 200 nm; (step 2) using the second type of detection to detect the eggs; and (step 3) processing detection results of both the first type of detection and the second type of detection for determining the weights.

12. A method according to any of the preceding claims, including, for classifying eggs:
- comparing at least the weights established according to the foregoing with criteria drawn up for classifying.

13. A method according to claim 12, including:
- in accordance with said classifying, sorting the eggs, wherein the eggs are collected in packing units.

14. A method according to claim 13, **characterized in that** the eggs are located on rotating rollers of an egg sorting machine.

## Patentansprüche

1. Verfahren zur Bestimmung der Gewichte von Eiern, umfassend
- das Aussetzen der Eier einer elektromagnetischen Strahlung mit einer Wellenlänge von mindestens 200 nm und dadurch Beleuchten und/oder Durchleuchten der Eier,
- das Aufzeichnen und Verarbeiten von Bildern, erhalten durch Unterschiede in Intensität oder Strahlung, übertragen während des Beleuchtens und/oder Durchleuchtens, wobei Bilddaten und/oder Derivate von diesen erstellt werden,
**dadurch gekennzeichnet, dass**
das Verfahren ferner mindestens eine zweite Art der Detektion umfasst, wobei in Kombination mit den Bilddaten und/oder den Derivaten von diesen das Gewicht jedes Eis erhalten wird.

2. Verfahren nach Anspruch 1, **dadurch gekennzeichnet, dass** die zweite Art der Detektion das Bestimmen von mindestens der Festigkeit K und/oder Derivaten davon der Eierschale von jedem Ei aufgrund eines Ergebnisses einer auf die Eierschale ausgeübten zeitlich variierenden Druckkraft auf die Eierschale umfasst.

3. Verfahren nach Anspruch 2, **dadurch gekennzeichnet, dass** ein solches Ei mit einer Klopfvorrichtung zum Vibrieren gebracht wird, wobei mindestens die Festigkeit K und/oder Derivate davon anhand der Vibrationseigenschaften des Eis bestimmt werden, wobei dann das Gewicht durch Kombinieren der Bilddaten und/oder der Derivate davon mit der Schalenfestigkeit und/oder den Derivaten davon bestimmt wird.

4. Verfahren nach Anspruch 2, **dadurch gekennzeichnet, dass** ein solches Ei mit einer Klopfvorrichtung zum Vibrieren gebracht wird, wobei mindestens die Festigkeit K und/oder Derivate davon anhand der Vibrationseigenschaften der Klopfvorrichtung bestimmt werden, wobei dann das Gewicht durch Kombinieren der Bilddaten und/oder der Derivate davon mit der Schalenfestigkeit und/oder den Derivaten davon bestimmt wird.

5. Verfahren nach Anspruch 1, **dadurch gekennzeichnet, dass** die zweite Art der Detektion umfasst, dass die Eier Röntgenstrahlen ausgesetzt werden, wobei mindestens Daten der Luftzelle des Eis erhalten werden, wobei das Gewicht durch Kombinieren der Bilddaten und/oder der Derivate davon mit den Daten der Luftzelle bestimmt wird.

6. Verfahren nach Anspruch 1, **dadurch gekennzeichnet, dass** die zweite Art der Detektion umfasst, dass die Eier Röntgenstrahlen ausgesetzt werden, wobei mindestens Daten der Eierschale von jedem Ei erhalten werden, wobei das Gewicht durch Kombinieren der Bilddaten und/oder der Derivate davon mit den Daten der Eierschale bestimmt wird.

7. Verfahren nach Anspruch 5 oder 6, **dadurch gekennzeichnet, dass** die Röntgenstrahlen eine Wellenlänge von mindestens 5 nm aufweisen, um damit die Eier zu durchleuchten,
- wobei Bilder von Unterschieden in der während des Durchleuchtens der Eier übertragenen Strahlungsintensität aufgezeichnet werden,
- wobei diese Bilder verarbeitet werden, wobei mindestens davon abgeleitete Übergänge und/oder Merkmale in wesentlicher Dichte ermittelt werden.

8. Verfahren nach einem der Ansprüche 5, 6 oder 7, **dadurch gekennzeichnet, dass** die Röntgenstrahlung im Wesentlichen Strahlung des Linienspektrumtyps umfasst.

9. Verfahren nach einem der Ansprüche 5, 6 oder 7, **dadurch gekennzeichnet, dass** die Röntgenstrahlung im Wesentlichen Strahlung des ununterbrochenen Strahlungstyps umfasst.

10. Verfahren nach einem der Ansprüche 5, 6 oder 7, **dadurch gekennzeichnet dass** die Röntgenstrahlung sowohl Strahlung des ununterbrochenen Spektrumtyps als auch des Linienspektrumtyps umfasst.

11. Verfahren nach einem der vorhergehenden Ansprüche, umfassend (Schritt 1) die Verwendung einer ersten Art von Eidetektion, umfassend das Erstellen der Bilder der Eier unter Verwendung der elektromagnetischen Strahlung mit einer Wellenlänge von mindestens 200 nm; (Schritt 2) die Verwendung der zweiten Art der Eidetektion; und (Schritt 3) die Verarbeitung der Detektionsergebnisse der ersten Detektionsart und der zweiten Detektionsart zur Bestimmung der Gewichte.

12. Verfahren nach einem der vorhergehenden Ansprüche, enthaltend, zur Klassifizierung von Eiern:
- Vergleichen von mindestens der nach dem vorhergehenden Verfahren ermittelten Gewichte mit Kriterien, die zur Klassifizierung erstellt wurden.

13. Verfahren nach Anspruch 12, enthaltend:
- in Übereinstimmung mit der Klassifizierung, das Sortieren der Eier, wobei die Eier in Verpackungseinheiten gesammelt werden.

14. Verfahren nach Anspruch 13, **dadurch gekennzeichnet, dass** sich die Eier auf drehenden Rollen einer Eiersortiermaschine befinden.

## Revendications

1. Procédé de détermination des poids des oeufs, comprenant :
- l'exposition des oeufs à un rayonnement électromagnétique ayant une longueur d'onde d'au moins 200 nm et, de ce fait, l'éclairage et/ou le mirage des oeufs,
- l'enregistrement et le traitement des images qui sont obtenues à partir des différences d'intensité du rayonnement émis pendant l'éclairage et/ou le mirage, moyennant quoi des données d'image et/ou des déductions de celles-ci sont établies,
**caractérisé en ce que**
le procédé comprend en outre au moins un deuxième type de détection,
dans lequel, en combinaison avec lesdites données d'image et/ou déductions de celles-ci, le poids de chaque oeuf est obtenu.

2. Procédé selon la revendication 1, **caractérisé en ce que** ledit deuxième type de détection comprend la détermination au moins de la rigidité K et/ou de déductions de celle-ci de la coquille d'oeuf de chaque oeuf en tant que résultat d'une force de pression variant dans le temps et exercée sur ladite coquille d'oeuf.

3. Procédé selon la revendication 2, **caractérisé en ce que** un tel oeuf est amené à vibrer par une unité de tapotement, dans lequel au moins ladite rigidité K et/ou lesdites déductions de celle-ci sont déterminées à partir des caractéristiques de vibration dudit oeuf, dans lequel, sur ce, le poids est déterminé en combinant lesdites données d'image et/ou déductions de celles-ci avec ladite rigidité de la coque et/ou lesdites déductions de celle-ci.

4. Procédé selon la revendication 2, **caractérisé en ce que**
un tel oeuf est amené à vibrer par une unité de tapotement, dans lequel au moins ladite rigidité K et/ou lesdites déductions de celle-ci sont déterminées à partir des caractéristiques de vibration de ladite unité de tapotement, dans lequel, sur ce, le poids est déterminé en combinant lesdites données d'image et/ou déductions de celles-ci avec ladite rigidité de la coque et/ou lesdites déductions de celle-ci.

5. Procédé selon la revendication 1, **caractérisé en ce que** ledit deuxième type de détection comprend l'exposition des oeufs à des rayons X, moyennant quoi au moins des données de la chambre à air de chaque oeuf sont obtenues, dans lequel le poids est déterminé en combinant lesdites données d'image et/ou déductions de celles-ci avec lesdites données de la chambre à air.

6. Procédé selon la revendication 1, **caractérisé en ce que** ledit deuxième type de détection comprend l'exposition des oeufs à des rayons X, moyennant quoi au moins des données de la coquille d'oeuf de chaque oeuf sont obtenues, dans lequel le poids est déterminé en combinant lesdites données d'image et/ou déductions de celles-ci avec lesdites données de la coquille d'oeuf.

7. Procédé selon la revendication 5 ou 6, **caractérisé en ce que**
les rayons X ont une longueur d'onde d'au plus 5 nm pour le mirage par ceux-ci des oeufs,
- dans lequel des images sont enregistrées avec des différences d'intensité du rayonnement émis pendant le mirage, et
- dans lequel ces images sont traitées, moyennant quoi au moins des transitions et/ou des caractéristiques déduites de celles-ci dans une densité substantielle sont établies.

8. Procédé selon l'une quelconque des revendications 5, 6, ou 7, **caractérisé en ce que** le rayonnement de rayons X comprend un rayonnement substantiel du type spectre de raies.

9. Procédé selon l'une quelconque des revendications 5, 6, ou 7, **caractérisé en ce que** le rayonnement de rayons X comprend un rayonnement substantiel du type spectre continu.

10. Procédé selon l'une quelconque des revendications 5, 6, ou 7, **caractérisé en ce que** le rayonnement de rayons X comprend un rayonnement à la fois du type spectre continu et spectre de raies.

11. Procédé selon l'une quelconque des revendications précédentes, comprenant (étape 1) l'utilisation d'un premier type de détection d'oeuf, comprenant l'obtention des images des oeufs en utilisant le rayonnement électromagnétique ayant une longueur d'onde d'au moins 200 nm ; (étape 2) l'utilisation du deuxième type de détection pour détecter les oeufs ; et (étape 3) le traitement des résultats de détection à la fois du premier type de détection et du deuxième type de détection pour déterminer les poids.

12. Procédé selon l'une quelconque des revendications précédentes, comprenant, pour classifier les oeufs,
- la comparaison au moins des poids établis selon ce qui précède avec des critères établis pour la classification.

13. Procédé selon la revendication 12, comprenant :
- conformément à ladite classification, le tri des oeufs, dans lequel les oeufs sont collectés dans des unités de conditionnement.

14. Procédé selon la revendication 13, **caractérisé en ce que**
les oeufs sont situés sur des rouleaux rotatifs d'une machine de tri d'oeufs.
